# EUROPÄISCHE PATENTSCHRIFT

(11) **EP 2 974 733 B1**
(45) Veröffentlichungstag und Bekanntmachung des Hinweises auf die Patenterteilung: **07.09.2016**
(21) Anmeldenummer: 14176927.3
(22) Anmeldetag: 14.07.2014
(51) Int. Cl.: A61K 36/84, A61K 36/53, A61P 25/00, A61P 25/20

(54) **Kombination von Baldrianwurzelextrakt und Lavendelöl zur Verwendung zur Behandlung von Schlafstörungen**
Combination of valerian root extract and lavender oil for use in the treatment of sleep disorders
Combinaison d'extrait de racine de valériane et d'huile de lavande destinée à être utilisée pour le traitement des troubles du sommeil

(43) Veröffentlichungstag der Anmeldung: 20.01.2016
(73) Patentinhaber: Dr. Willmar Schwabe GmbH & Co. KG, 76227 Karlsruhe (DE)
(72) Erfinder: DIMPFEL, Wilfried, 35578 Wetzlar (DE); NÖLDNER, Michael, 76139 Karlsruhe (DE)
(74) Vertreter: Adam, Holger

(56) Entgegenhaltungen:
- US-A1- 2009 061 029
- DATABASE WPI Week 201379 Thomson Scientific, London, GB; AN 2013-M61002 XP002728910, & KR 101 296 920 B1 (BOK L G) 14. August 2013 (2013-08-14)
- BASCH E ET AL: "LAVENDER (LAVANDULA ANGUSTIFOLIA MILLER)", JOURNAL OF HERBAL PHARMACOTHERAPY, HAWORTH HERBAL PRESS, BINGHAMTON, US, Bd. 4, Nr. 2, 1. Januar 2004 (2004-01-01), Seiten 63-78, XP009058904, ISSN: 1522-8940, DOI: 10.1300/J157V04N02_07
- FISSLER MARIA ET AL: "A case series on the use of lavendula oil capsules in patients suffering from major depressive disorder and symptoms of psychomotor agitation, insomnia and anxiety", COMPLEMENTARY THERAPIES IN MEDICINE, Bd. 22, Nr. 1, 14. August 2013 (2013-08-14), Seiten 63-69, XP028616779, ISSN: 0965-2299, DOI: 10.1016/J.CTIM.2013.11.008
- KALYANAKRISHNAN RAMAKRISHNAN ET AL: "Treatment options for insomnia", AMERICAN FAMILY PHYSICIAN, Bd. 76, Nr. 4, 15. August 2007 (2007-08-15), Seiten 517-528, XP55136407, US ISSN: 0002-838X
- BRAUN L: "Herbs for insomnia", AUSTRALIAN JOURNAL OF PHARMACY 200810 AU, Bd. 89, Nr. 1062, Oktober 2008 (2008-10), Seiten 70-71, XP009179800, ISSN: 0311-8002
- KRAFT K: "Schlaf- und psychische Störungen im Kindesalter = Sleep and psychiatric disorders in childhood", ZEITSCHRIFT FUER PHYTOTHERAPIE, HIPPOKRATES VERLAG IN MVS MEDIZINVERLAGE, DE, Bd. 28, Nr. 5, 1. Januar 2007 (2007-01-01) , Seiten 235-237, XP009151133, ISSN: 0722-348X, DOI: 10.1055/S-2007-992170
- CHUNG-SOO KIM ET AL: "Herbs for the Treatment of Insomnia", BIOMOLECULES & THERAPEUTICS, Bd. 19, Nr. 3, 31. Juli 2011 (2011-07-31), Seiten 274-281, XP55134372, KR ISSN: 1976-9148, DOI: 10.4062/biomolther.2011.19.3.274
- DATABASE WPI Week 201454, Derwent Publications Ltd., London, GB; AN 2014-L88082 & CN 103 750 535 A (HONGYUN HONGHE TOBACCO GROUP CO LTD) 30 April 2014
- DATABASE WPI Week 199844, Derwent Publications Ltd., London, GB; AN 1998-518894 & RU 2 106 873 C1 (BOBROV-EGOROV N N) 20 März 1998

## Beschreibung

Die vorliegende Erfindung betrifft die Verwendung einer Kombination von Baldrianwurzelextrakt (Extrakt aus getrockneten Wurzeln von Valeriana officinalis) und Lavendelöl (Öl aus Blüten von Lavandula officinalis) zur oralen Behandlung von Schlafstörungen.

Die vorliegende Erfindung betrifft die Verwendung einer Kombination von Lavendelöl und Baldrianwurzelextrakt als hochpotentes Schlafmittel. Die Kombination führt zu einer synergistischen, überadditiven Wirksamkeit bezüglich der Induktion und der Tiefe von Schlaf.

Lavendelöl wird zu verschiedenen Zwecken hergestellt und beispielsweise zur Aromatherapie verwendet. Dabei wird ihm traditionell eine beruhigende Wirkung zugeschrieben. Eine spezielle Zubereitung zur oralen Einnahme ist seit einigen Jahren als Arzneimittel in Deutschland zur Behandlung von Unruhezuständen bei ängstlicher Verstimmung (Lasea^{®} der Firma Dr. Willmar Schwabe GmbH & Co. KG) zugelassen. Baldrianwurzelextrakt wird traditionell bei nervös bedingten Einschlafstörungen verwendet und existiert ebenfalls als Medikament (z.B. Baldiparan^{®} Stark für die Nacht der Firma Pfizer Consumer Healthcare GmbH).

Über mögliche biologische oder pharmakologische Wirkungen von Lavendelöl zur Behandlung von Schlafstörungen wird zwar berichtet, für diese Anwendung gibt es bisher aber keine gesicherten klinischen Studien. Eine Kombination mit Baldrianwurzelextrakt zur Verbesserung des Schlafes wurde bisher noch nicht beschrieben. Umso überraschender war der Befund, dass Lavendelöl in der Lage ist, bei äußerst schwacher Eigenwirkung, die Wirkung von Baldrianwurzelextrakt extrem zu verstärken bzw. zu potenzieren. Hinweise auf das Vorhandensein einer derartigen überadditiven, potenzierenden Wirkung erfolgten bei Untersuchungen im Tele-Stereo-EEG der Ratte im Rahmen von Versuchen mit anderer Zielsetzung.

Die derzeit üblichen Arzneimittel zur Behandlung von Schlafstörungen haben ein breites Nebenwirkungsspektrum (Abhängigkeit und Toleranzentwicklung bei den Benzodiazepinen).

Es besteht daher ein großer Bedarf nach verbesserten Behandlungsmöglichkeiten für Schlafstörungen, sei es als Arzneimittel oder als Lebensmittel mit einer guten Wirksamkeit bei möglichst geringer Nebenwirkungsrate. Diese geringe Nebenwirkungsrate ist bei Verabreichung eines Naturstoffs oder einer Kombination von Naturstoffen eher zu erwarten.

Diese Aufgabe wurde überraschenderweise durch die Verwendung von Lavendelöl in Kombination mit dem Extrakt der Baldrianwurzel gelöst.

Die Erfindung betrifft die Kombination umfassend einen Gehalt an Baldrianwurzelextrakt (Extrakt aus getrockneten Wurzeln von Valeriana officinalis) und Lavendelöl (Öl aus Blüten von Lavandula officinalis) zur oralen Verwendung bei der Behandlung oder Prophylaxe von Schlafstörungen, wobei der Baldrianwurzelextrakt durch Extraktion mit wässrigem Ethanol oder wässrigem Methanol hergestellt ist, wobei Ethanol und Wasser im Volumenverhältnis von 30/70 bis 90/10 oder Methanol und Wasser im Volumenverhältnis von 40/60 bis 55/45 bei der Extraktion eingesetzt werden. Die Kombination kann optional weitere pflanzliche Naturstoffe oder Extrakte umfassen, umfasst vorzugsweise aber keine weiteren Pflanzenextrakte und/oder Pflanzenöle und/oder sonstige arzneilich wirksame Stoffe (Wirkstoffe). Die Kombination umfassend Baldrianwurzelextrakt und Lavendelöl zur Verwendung bei der Behandlung oder Prophylaxe von Schlafstörungen kann in Form einer Darreichungsform vorliegen, d.h. in Form eines Medikamentes oder Lebensmittels oder kann in Form zweier separater Darreichungsformen vorliegen, d.h. in Form zweier separater Medikamente oder Lebensmittel, die jeweils den Baldrianwurzelextrakt bzw. das Lavendelöl enthalten. Für den Fall, dass der Baldrianwurzelextrakt und das Lavendelöl jeweils in separaten Darreichungsformen in z.B. Form eines Medikamentes oder Lebensmittels vorliegen, können diese zeitgleich miteinander oder zeitlich nacheinander in beliebiger Reihenfolge, d.h. zuerst der Baldrianwurzelextrakt und dann das Lavendelöl oder zuerst das Lavendelöl und dann der Baldrianwurzelextrakt verabreicht werden. Die zeitliche Folge der Verabreichung sollte nicht länger als eine Stunde, vorzugsweise nicht länger als 30 Minuten und insbesondere nicht länger als 10 Minuten betragen. Vorzugsweise werden auch bei separater Verabreichung der Baldrianwurzelextrakt und das Lavendelöl zeitgleich oder nahezu zeitgleich, d.h. binnen weniger Minuten (bis zu 10 Minuten) verabreicht.

Die nachfolgenden Ausführungen beziehen sich auf die erfindungsgemäße orale Verwendung der Kombination umfassend Baldrianwurzelextrakt und Lavendelöl und gegebenenfalls pharmazeutisch bzw. lebensmittelrechtlich akzeptable Hilfsstoffe, d.h. in einem Arzneimittel bzw. Lebensmittel.

In vivo Versuche an der Ratte zeigen Ergebnisse, die die Überlegenheit einer Kombination von Lavendelöl mit einem Extrakt der Baldrianwurzel belegen. Die überraschend gefundene überadditive, synergistische Wirkung wurde in vivo an der Ratte dokumentiert und in der Folge an einzelnen Patienten mit Schlafstörungen verifiziert.

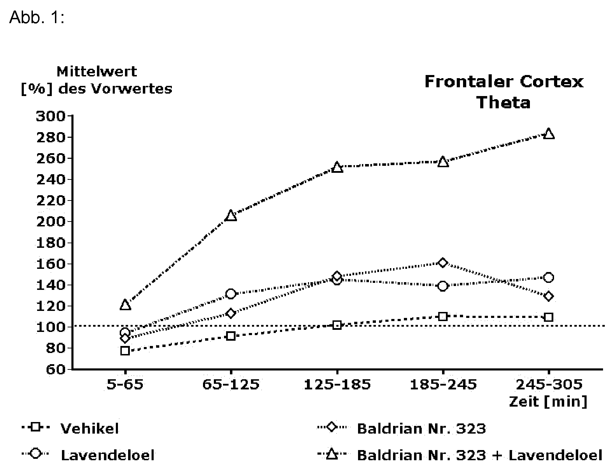

Kurze Beschreibung der Abbildungen
Abb. 1 Zeitlicher Verlauf der theta Wellen über 5 Stunden nach Verabreichung der einzelnen Komponenten (80 mg/kg Baldrianwurzelextrakt; 80 mg/kg Lavendelöl) im Vergleich mit der Kombination von Baldrianwurzelextrakt und Lavendelöl (80 mg/kg + 80 mg/kg) bei der Ratte. Die γ-Achse zeigt die Leistungsdichte in % der Änderung vom 45-minütigen Vorwert vor Verabreichung. Man beachte, dass beide Komponenten jeweils allein verabreicht in der ersten Stunde noch keinerlei Wirkung auf diese Frequenz haben, jedoch in Kombination bereits wirksam sind. Die synergistische Wirkung ist überadditiv.

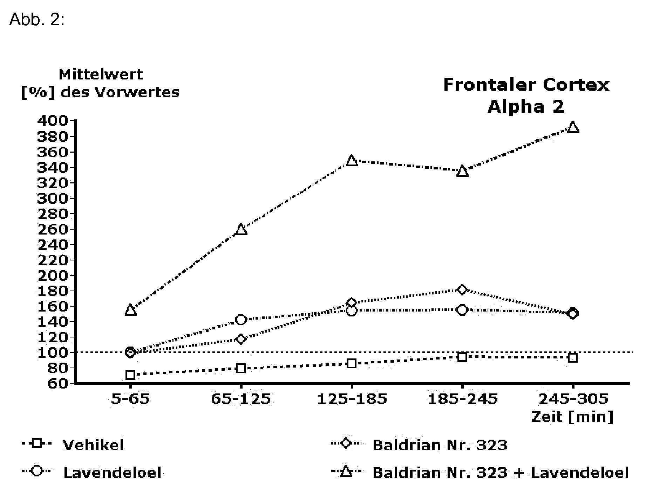

Abb. 2 Zeitlicher Verlauf der alpha 2 Wellen über 5 Stunden nach Verabreichung der einzelnen Komponenten (80 mg/kg Baldrianwurzelextrakt; 80 mg/kg Lavendelöl) im Vergleich mit der Kombination von Baldrianwurzelextrakt und Lavendelöl (80 mg/kg + 80 mg/kg) bei der Ratte. Die γ-Achse zeigt die Leistungsdichte in % der Änderung vom 45-minütigen Vorwert vor Verabreichung. Wie bereits für die theta Wellen beschrieben entwickelt sich eine überadditive Wirkung von der ersten Stunde nach oraler Gabe an.

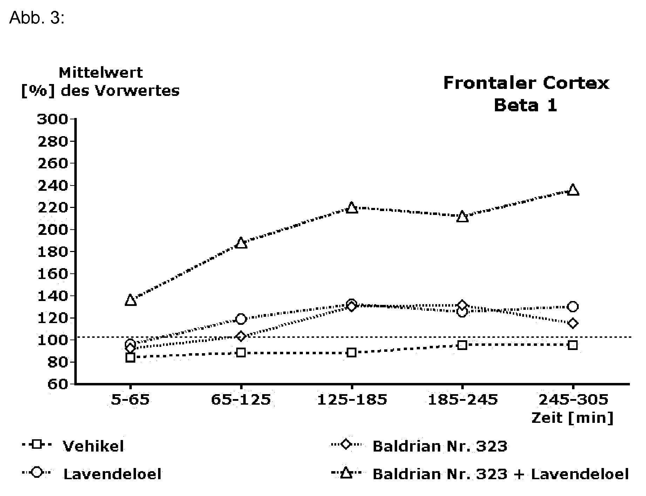

Abb. 3 Zeitlicher Verlauf der beta1 Wellen über 5 Stunden nach Verabreichung der einzelnen Komponenten (80 mg/kg Baldrianwurzelextrakt; 80 mg/kg Lavendelöl) im Vergleich mit der Kombination von Baldrianwurzelextrakt und Lavendelöl (80 mg/kg + 80 mg/kg) bei der Ratte. Die γ-Achse zeigt die Leistungsdichte in % der Änderung vom 45-minütigen Vorwert vor Verabreichung. Wie bereits für die theta und alpha2 Wellen beschrieben entwickelt sich eine überadditive Wirkung von der ersten Stunde nach oraler Gabe an.

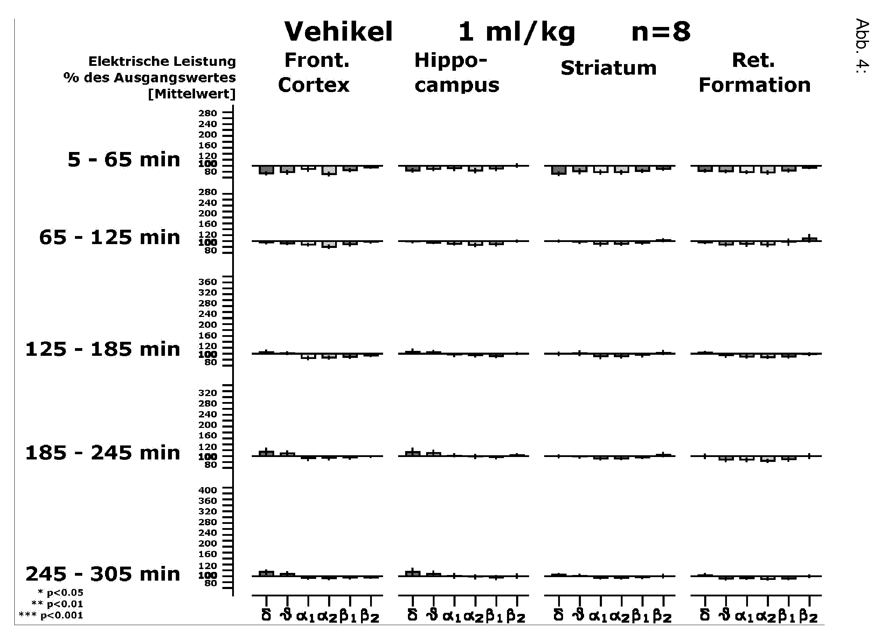

Abb. 4 Zeitlicher Verlauf der elektrischen Leistung aller Frequenzbereiche über 5 Stunden nach Verabreichung von Vehikel (Methylcellulose) in vier Hirnregionen: Frontaler Kortex, Hippocampus, Striatum und Formatio Reticularis. Die sechs Frequenzbereiche delta (δ), theta (θ), alpha1 (α1), alpha2 (α2), beta1 (β1) und beta2 (β2) sind auf der Abszisse dokumentiert.

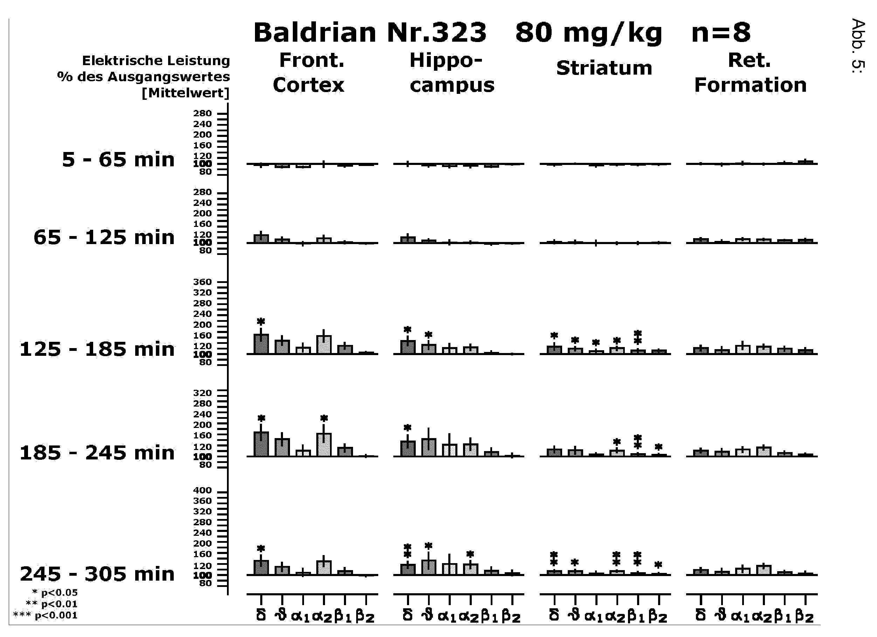

Abb. 5 Zeitlicher Verlauf der elektrischen Leistung aller Frequenzbereiche über 5 Stunden nach Verabreichung von 80 mg/kg Baldrianwurzelextrakt allein in vier Hirnregionen: Frontaler Kortex, Hippocampus, Striatum und Formatio Reticularis. Die sechs Frequenzbereiche delta (δ), theta (θ), alpha1 (α1), alpha2 (α2), beta1 (β1) und beta2 (β2) sind auf der Abszisse dokumentiert. Die statistische Signifikanz im Vergleich zu Kontrolle (Vehikel) ist durch Sterne dokumentiert: *=p<0.05; **=p<0.01.

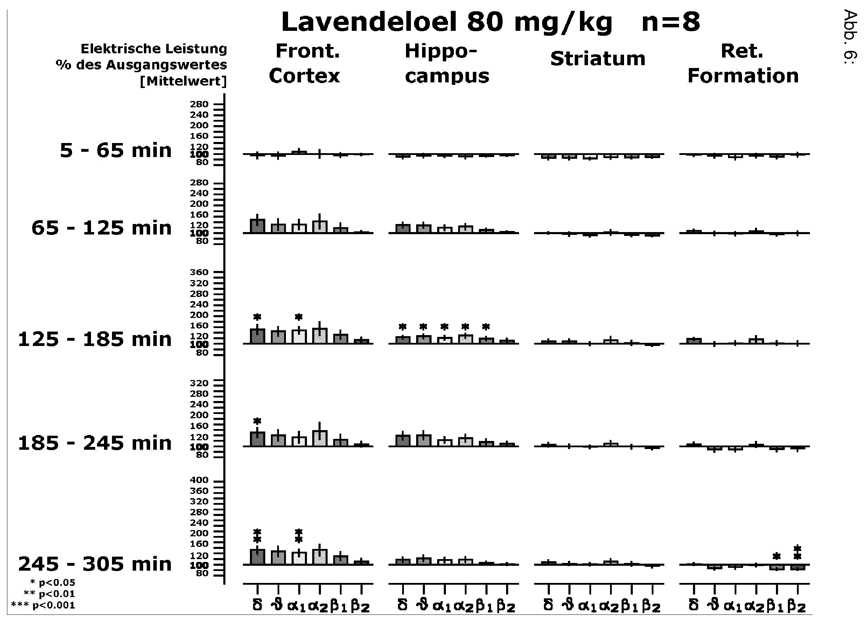

Abb. 6 Zeitlicher Verlauf der elektrischen Leistung aller Frequenzbereiche über 5 Stunden nach Verabreichung von 80 mg/kg Lavendelöl allein in vier Hirnregionen: Frontaler Kortex, Hippocampus, Striatum und Formatio Reticularis. Die sechs Frequenzbereiche delta (δ), theta (θ), alpha1 (α1), alpha2 (α2), beta1 (β1) und beta2 (β2) sind auf der Abszisse dokumentiert. Die statistische Signifikanz im Vergleich zu Kontrolle (Vehikel) ist durch Sterne dokumentiert: *=p<0.05; **=p<0.01.

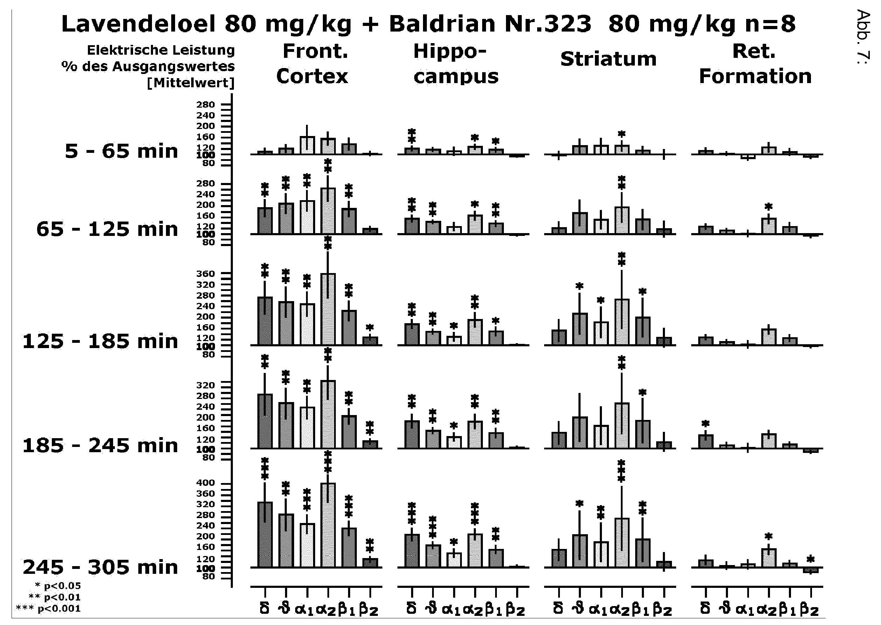

Abb. 7 Zeitlicher Verlauf der elektrischen Leistung aller Frequenzbereiche über 5 Stunden nach Verabreichung der Kombination von 80 mg/kg Baldrianwurzelextrakt und 80 mg/kg Lavendelöl in vier Hirnregionen: Frontaler Kortex, Hippocampus, Striatum und Formatio Reticularis. Die sechs Frequenzbereiche delta (δ), theta (θ), alpha1 (α1), alpha2 (α2), beta1 (β1) und beta2 (β2) sind auf der Abszisse dokumentiert. Die statistische Signifikanz im Vergleich zu Kontrolle (Vehikel) ist durch Sterne dokumentiert: *=p<0.05; **=p<0.01; ***=p<0.001.

Bei Untersuchungen der Veränderungen der EEG-Frequenzen der Ratte wurde überraschenderweise im Vergleich zur alleinigen Gabe von Baldrianwurzelextrakt eine deutlich stärkere Zunahme der spektralen Leistung nach Kombination mit Lavendelöl gefunden, die bei einer Behandlung von Schlafstörungen nützlich ist, da die Zunahme dieser Frequenzen mit der Schlaftiefe korreliert. Die alleinige Gabe von Vehikel verursachte keine Änderungen der elektrischen Leistung (Abb. 4).

Die Abb. 1 zeigt die zeitabhängigen Veränderungen der theta EEG Frequenzen nach oraler Verabreichung verschiedener Dosierungen von Lavendelöl oder Baldrianwurzelextrakt allein sowie als Kombination. In früheren Versuchen konnte gezeigt werden, dass vor allem die theta und beta1 Wellen des EEG die Schlaftiefe repräsentieren (Dimpfel W, Hofmann H-C Mehrdimensionale Dokumentation des Schlafes auf der Grundlage der Frequenzanalyse. In Mayer G (Hrgs). Jahrbuch Schlafmedizin Deutschland 1994: MMV Verlag, München). Obwohl Lavendelöl bei alleiniger Gabe kaum eine Erhöhung der theta Frequenzen bewirkt, ist zu erkennen, dass die trotz hoher Dosis ebenfalls nur geringe Wirkung von Baldrianwurzelextrakt massiv überboten wird und damit eine größere Schlaftiefe erreicht wird. Auch die alpha2 Frequenzen verändern sich in analoger Weise (Abb. 2). Ähnliches trifft für die für die Schlaftiefe wichtige beta1 Frequenz zu. Wie in Abb. 3 dargestellt übertrifft die Wirkung der Kombination die Wirkung beider Einzelpräparate deutlich, wobei sowohl Baldrianwurzelextrakt als auch Lavendelöl allein kaum eine Wirkung erkennen lässt. Diese synergistische Wirkung konnte bei einzelnen Schlaf-gestörten Patienten bestätigt werden. Diese Ergebnisse zeigen, dass eine Kombination von Baldrianwurzelextrakt und Lavendelöl zu einem hochpotenten Naturheilmittel zur Behandlung von Schlafstörungen mit geringem Nebenwirkungspotential führt.

In einer bevorzugten Ausführungsform sind die Schlafstörungen ausgewählt aus Ein- und Durchschlafstörungen (G47.0 gemäß ICD-10), Störungen des Schlaf-Wach-Rhythmus (G47.2 gemäß ICD-10), Schlafapnoe (G47.3 gemäß ICD-10), nichtorganischer Insomnie (F 51.0 gemäß ICD-10), nichtorganische Störung des Schlaf-Wach-Rhythmus (F51.2 gemäß ICD-10), Schlafwandeln (Sonambulismus, F51.3 gemäß ICD-10), Pavor nocturnus (F51.4 gemäß ICD-10) sowie Alpträume (Angstträume, F51.5 gemäß ICD-10).

Erfindungsgemäß wird Lavendelöl in Kombination mit Baldrianwurzelextrakt eingesetzt. Dabei können die beiden Komponenten Lavendelöl und Baldrianwurzelextrakt als Mischung in einer Darreichungsform (Kombinationspräparat) oder als separate Darreichungsformen appliziert werden. In beiden Fällen können zusätzlich pharmazeutisch bzw. lebensmittelrechtlich akzeptable Hilfsstoffe enthalten sein. In einer bevorzugten Ausführungsform sind in der Kombination keine weiteren Pflanzenextrakte und/oder Pflanzenöle und/oder sonstige arzneilich wirksame Stoffe (Wirkstoffe) enthalten.

Der in der erfindungsgemäß verwendeten Kombination enthaltene Baldrianwurzelextrakt wird bevorzugt aus getrockneten Wurzeln von Valeriana officinalis hergestellt. Die Herstellung des Extraktes erfolgt mit wässrigem Ethanol oder mit wässrigem Methanol als Extraktionslösungsmittel, wobei Ethanol und Wasser im Volumenverhältnis von 30/70 bis 90/10 oder Methanol und Wasser im Volumenverhältnis von 40/60 bis 55/45 eingesetzt werden.

In einer bevorzugten Ausführungsform wird als Baldrianwurzelextrakt ein Baldrianwurzel-trockenextrakt eingesetzt. (Gemäß Europäischem Arzneibuch weist ein Trockenextrakt einen Trocknungsverlust bzw. einen Wassergehalt von weniger als 5% auf). Der Baldrianwurzeltrockenextrakt enthält vorzugsweise mindestens 0,25 Gew.-% Sesquiterpensäuren (Summe aus Valerensäure, Hydroxyvalerensäure und Acetoxyvalerensäure, berechnet als Valerensäure) und entspricht damit den Vorgaben des Europäischen Arzneibuchs, Ausgabe 8.0. In einer weiteren bevorzugten Ausführungsform beträgt dabei das Gewichtsverhältnis von Valerensäure zu Acetoxyvalerensäure mindestens 2/1.

Das in obiger erfindungsgemäß verwendeter Kombination enthaltene Lavendelöl wird bevorzugt gemäß der Monographie des Europäischen Arzneibuchs, Ausgabe 8.0 "Lavender Oil" durch Wasserdampfdestillation aus Blüten von Lavandula officinalis gewonnen und bei Bedarf durch eine weitere Destillation aufgereinigt. In einer bevorzugten Ausführungsform enthält das Lavendelöl 20,0 Gew.-% bis 45,0 Gew.-% Linalool und 25,0 Gew.-% bis 47,0 Gew.-% Linalylacetat sowie höchstens 1,0 Gew.-% Limonen, höchstens 2,5 Gew.-% 1,8-Cineol, 0,1 Gew.-% bis 5,0 Gew.-% 3-Octanon, höchstens 1,2 Gew.-% Campher, 0,1 Gew.-% bis 8,0 Gew.-% Terpinen-4-ol, mindestens 0,2 Gew.-% Lavandulylacetat, mindestens 0,1 Gew.-% Lavandulol und höchstens 2,0 Gew.-% alpha-Terpineol und entspricht damit den Vorgaben des Europäischen Arzneibuchs, Ausgabe 8.0..

Lavendelöl und Baldrianwurzelextrakt können erfindungsgemäß auch in Form hochgereinigter Präparate eingesetzt werden. Die Herstellung solcher Präparate erfolgt in an sich bekannter Weise.

Die Lavendelöl in Kombination mit Baldrianwurzelextrakt als wirksame Komponente enthaltenden Arzneimittel bzw. Lebensmittel (Kombinationspräparate) bzw. die separaten Darreichungsformen, die jeweils nur Lavendelöl oder Baldrianwurzelextrakt als wirksame Komponente enthalten, werden oral verabreicht.

Zur Verabreichung kann das Arzneimittel bzw. Lebensmittel, das als wirksame Komponente die Kombination von Baldrianwurzelextrakt und Lavendelöl oder den Baldrianwurzelextrakt bzw. das Lavendelöl separat enthält, z.B. als feste Darreichungsform (z.B. Tablette, Kapsel, Dragee, Pellet, Granulat, Suppositorium) oder flüssige Darreichungsform (Lösung, Emulsion, Suspension) formuliert werden, wobei der Wirkstoff optional mit pharmazeutisch annehmbaren Hilfsund Trägerstoffen kombiniert werden kann.

Liegt das Arzneimittel bzw. Lebensmittel in oral applizierbarer Form vor, so enthält es bevorzugt eine Kombination von Baldrianwurzelextrakt und Lavendelöl im Gewichtsverhältnis von 12,5:1 bis 1:2,5, vorzugsweise 7:1 bis 1:1. Andere Mischungsverhältnisse sind möglich.

Die Arzneimittel bzw. Lebensmittel, die Lavendelöl und Baldrianwurzelextrakt als wirksame Komponenten enthalten, werden normalerweise gemäß herkömmlichen Methoden hergestellt und in einer pharmazeutisch geeigneten Form verabreicht.

Die festen Darreichungsformen können neben den Wirkstoffen (wirksamen Komponenten) in der Pharmazie und der Lebensmitteltechnologie üblicherweise verwendete Hilfsstoffe enthalten. Häufig verwendete Füllmittel sind z.B. Zucker (Laktose, Sucrose), Zuckeralkohole (Mannitol, Sorbitol, Maltitol, Xylitol, Isomaltol), Stärke und Cellulose. Zusätzlich können weitere technologisch erforderliche Hilfsstoffe enthalten sein, wie z.B. Bindemittel (Gelatine, Gummi arabicum, Cellulose, Methylcellulose, Hydroxypropylcellulose, Polyvinylpyrrolidon), Fließregulierungsmittel (hochdisperses Siliciumdioxid, gefälltes Siliciumdioxid), Zerfallsbeschleuniger (Croscarmellose, Crospovidon), Schmiermittel (Magnesiumstearat, Calciumstearat, Stearinsäure, Glycerylbehenat).

Zur Verbesserung der Einnahme und zur Abdeckung eines unangenehmen Geschmacks oder Geruchs können die festen Formen (z.B. Tabletten) mit üblicherweise verwendeten Überzugsmitteln beschichtet sein. Zum Beschichten finden Lösungen von Zuckern oder Zuckeralkoholen Verwendung, es können also Dragees hergestellt werden. Alternativ können die festen Formen mit einem Polymerfilm überzogen, also Filmtabletten hergestellt werden. Diese Filme bestehen üblicherweise aus einem Überzugspolymer (z.B. Hydroxypropylmethylcellulose), Weichmacher (z.B. Polyethylenglycol, Glycerol, Triethylcitrat, Glyceroltriacetat), Antiklebemittel (z.B. Talkum, Glycerolmonostearat), Entschäumer (Polydimethylsiloxan) und organischen oder anorganischen Farbpigmenten (z.B. Eisenoxid, Titandioxid, Carmin).

Zur Verhinderung eines zu schnellen Zerfalls der festen Darreichungsform nach der Einnahme, verbunden mit einem unangenehmen Geschmacks- oder Geruchsempfinden durch vermehrtes Aufstoßen, kann die feste Darreichungsform mit einem Überzug versehen werden, der den Zerfall im Magen verhindert und eine Freisetzung der Wirkstoffe im Darm bewirkt. Üblicherweise werden zu diesem Zweck Magensaft-unlösliche, Darmsaft-lösliche Überzugspolymere wie z.B. Poly(methacrylsäure-co-ethylacrylat) oder Schellack verwendet.

Eine weitere bevorzugte Ausführungsform der Erfindung besteht in der Verwendung von Kapseln als Darreichungsform. Kapseln haben den Vorteil, dass sehr unterschiedliche Stoffe in fester oder flüssiger Form in variablen Mengen abgefüllt werden können. Bei den genannten Kapseln gibt es zwei verschiedene Ausführungsformen und zwar Hartkapseln, die aus zwei Teilen bestehen, die nach der Abfüllung zum Verschließen zusammengesteckt werden und Weichkapseln, die in einem Arbeitsgang geformt, befüllt und verschlossen werden. Hartkapseln bestehen üblicherweise aus Gelatine oder modifizierter Stärke und sind für die Befüllung mit festen oder flüssigen Füllgütern geeignet. Im letzteren Fall wird die Kapselnaht nach dem Verschließen mit einer Banderole versehen, um das Austreten des Füllgutes zu verhindern. Weichkapseln bestehen üblicherweise aus Gelatine oder einer Mischung aus Carrageenan und modifizierter Stärke und werden mit flüssigen Füllgütern in Form von Lösungen, Emulsionen oder Suspensionen befüllt.

Die flüssigen Dispersionen zur oralen Verabreichung können z.B. Sirupe, Emulsionen und Suspensionen sein.

Der Sirup kann neben Wasser als Lösungsmittel Zucker (z.B. Sucrose), Zuckeralkohole (z.B. Sorbitol, Mannitol, Xylitol), Lösungsvermittler (Glycerol, Propylenglycol) und weitere Hilfsstoffe (Konservierungsmittel, Säuerungsmittel, Aromastoffe) enthalten.

Die Dosierung des Arzneimittels bzw. Lebensmittels beträgt bevorzugt 80 bis 600 mg Baldrianwurzelextrakt, besonders bevorzugt 250 bis 500 mg, in Kombination mit 40 bis 200 mg, besonders bevorzugt 40 bis 80 mg Lavendelöl pro Einzeldosis. Die Einzeldosis kann beispielsweise ein- bis dreimal, vorzugsweise ein- bis zweimal täglich verabreicht werden. Eine bevorzugte Kombination umfasst 250 bis 500 mg Baldriantrockenextrakt und 40 bis 80 mg Lavendelöl pro Einzeldosis. Eine besonders bevorzugte Kombination umfasst 500 mg Baldriantrockenextrakt und 80 mg Lavendelöl bzw. 250 mg Baldriantrockenextrakt und 40 mg Lavendelöl.

Bei Verwendung weiterer Extrakte muss die verwendete Menge in dem Fachmann bekannter Weise entsprechend angepasst werden.

Die erfindungsgemäß verwendete Kombination kann sowohl als Arzneimittel als auch als
Lebensmittel verwendet werden. Unter Lebensmittel sind hierbei insbesondere diätetische Lebensmittel, Nahrungsergänzungsmittel sowie "medical food" und "dietary supplements" zu verstehen.

### Testverfahren

### 1. Pentobarbital-induzierte Schlafzeit an der Maus

Um den Einfluss von Lavendelöl (hergestellt gemäß Beispiel 2), Baldrianwurzelextrakt (hergestellt gemäß Beispiel 1) bzw. der Kombination der beiden Substanzen auf den Schlaf von Mäusen zu überprüfen wurde das Modell der Pentobarbital-induzierten Schlafzeit ausgewählt. Dabei werden die Tiere mit den Prüfsubstanzen behandelt und anschließend wird Pentobarbital appliziert um zu messen, ob die induzierte Schlafzeit verändert wird. Sedierende Prüfsubstanzen verlängern die Pentobarbital-induzierte Schlafzeit.

Die Veränderungen der Schlafzeit wurden nach Gabe von Agarosegel (Kontrolle) bzw. von Lavendelöl, Baldrianwurzelextrakt oder Lavendelöl und Baldrianwurzelextrakt in Kombination bestimmt.

32 männliche NMRI Mäuse des Züchters Janvier, LaGenest, Frankreich wurden nach einer Eingewöhnungszeit von mindestens einer Woche für die Untersuchungen verwendet.Der eigentliche Versuch begann mit der oralen Behandlung der Mäuse mit den Kontroll- oder Prüfsubstanzen (Lavendelöl oder Baldrianwurzelextrakt oder die Kombination aus beiden). Eine Stunde später erhielten alle Versuchstiere eine intraperitoneale Applikation von 45 mg/kg Pentobarbital zur Schlafinduktion. Die Mäuse wurden anschließend in regelmäßigen Abständen beobachtet und wenn erkennbar wurde, dass die Tiere sediert sind, wurde der Stellreflex überprüft (die Tiere werden auf den Rücken gelegt und wenn sie liegen bleiben, wird dies als Schlaf/Sedation definiert) und die Zeit des Einschlafens dokumentiert. Wenn die Sedation nachlässt, und die Tiere erwachen, drehen sie sich wieder in Bauchlage und dieser als Aufwachzeit definierte Zeitpunkt wurde ebenfalls dokumentiert. Die Zeit vom Einschlafen bis zum Erwachen ist die Schlafzeit. Zwei Stunden nach individuellem Schlafbeginn wurde der Versuch beendet. Bei Tieren, die länger schlafen wurde die Schlafdauer auf 2 Stunden gesetzt.

Die Gabe von Baldrianwurzelextrakt oder Lavendelöl allein führt nicht zu Veränderungen der Pentobarbital-induzierten Schlafzeit. Die Gabe von Baldrianwurzelextrakt und Lavendelöl führt dagegen zu einer hochsignifikanten Verlängerung der Schlafzeit (Tabelle 1).

**Tabelle 1: Pentobarbital-induzierte Schlafzeit nach Gabe von Agarosegel (Kontrolle) bzw. von Lavendelöl, Baldrianwurzelextrakt oder Lavendelöl und Baldrianwurzelextrakt in Kombination**

| Substanz | Dosis mg/kg | Schlafzeit (Minuten) |
|---|---|---|
| Kontrolle | | 46,9 ± 21 |
| Baldrianwurzelextrakt | 80 | 38,4 ± 27 |
| Lavendelöl | 80 | 57,4 ± 23 |
| Baldrianwurzelextrakt + Lavendelöl | 80 + 80 | 90,8 ± 32 # |

| | | |
|---|---|---|
| # p < 0,01 T-Test | | |

### 2. Tele-Stereo-EEG an der Ratte

Die Veränderungen der EEG-Frequenzen wurde nach Gabe von Methylcellulose (Kontrolle) bzw. von Lavendelöl (hergestellt gemäß Beispiel 2), Baldrianwurzelextrakt (hergestellt gemäß Beispiel 1) oder Lavendelöl und Baldrianwurzelextrakt in Kombination bestimmt.

Die Untersuchungen wurden analog der durch W. Dimpfel beschriebenen Methode (Dimpfel W: Preclinical data base of pharmaco-specific rat EEG fingerprints (Tele-Stereo-EEG). Eur J Med Res (2003) 8: 199-207) folgendermaßen durchgeführt: Acht männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippocampus, Striatum und Formatio Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-Fourier-Transformation unterworfen und die Leistungsdichtespektren jeweils über 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Die Präparate wurden oral 45 Minuten nach Beginn der Messungen (Vorwert) appliziert. Fünf Minuten später wurden die Messungen wieder gestartet, mindestens über die nächsten 5 Stunden kontinuierlich analysiert und in 60-minütigen Perioden zusammengefasst. Die Testpräparate wurden in unterschiedlichen Kombinationen appliziert. Die experimentelle Serie wurde mit der Applikation von Methylcellulose (Kontrolle), die zu keiner auffälligen Änderung führte, begonnen.

Der statistische Vergleich der Versuche zu den Ergebnissen, die nach Gabe von Methylcellulose gemessen wurde, erfolgte nach Wilcoxon, Mann and Whitney auf der Basis der Veränderungen innerhalb der einzelnen Frequenzbänder in allen Hirnregionen als Variablen.

Die Verabreichung von Methylcellulose führte kaum zu Veränderungen der elektrischen Leistung (µV2) im Vergleich zu den Vorphasenwerten (Abb. 4). Die Verabreichung von Baldrianwurzelextrakt oder Lavendelöl alleine führte zu sehr schwachen, gerade messbaren Veränderungen der spektralen Leistung, die auch erst ab der zweiten Stunde nach Verabreichung erkennbar wurden (Abb. 1, 2, 3 sowie 5 und 6).

Die Verabreichung von Lavendelöl in Kombination mit Baldrianwurzelextrakt dagegen führte zu sehr frühen stabilen Veränderungen der Leistungsdichte bereits in der ersten Stunde nach Verabreichung vor allem im Kortex, vor allem aber auch während der dritten bis fünften Stunde nach Applikation. (siehe Abb. 1, 2, 3 und Abb. 7).

Die orale Verabreichung der Kombination von Baldrianwurzelextrakt und Lavendelöl als Einzeldosis führte zu extrem deutlichen und statistisch signifikanten Veränderungen der elektrischen Hirnaktivität bei den Testtieren, die denen während des Schlafes entsprachen. Insbesondere ist dies überraschend, da die Präparate am Morgen, d.h. nach der Nachtruhe (inaktive Phase) verabreicht wurden.

### Beispiel 1: Herstellung eines Baldrianwurzelextraktes gemäß Ph. Eur.

Getrocknete Wurzeln von Valeriana officinalis wurden zweimal mit der siebenfachen Gewichtsmenge 70 % (v/v) Ethanol bei 60 °C 1 h extrahiert. Die abgekühlte Mischung wurde filtriert. Das Filtrat wurde konzentriert und anschließend bei 60 °C und 25 mbar 8 h getrocknet: 24 % Ausbeute an Trockenextrakt.

### Beispiel 2: Herstellung eines Lavendelöls gemäß Ph. Eur.

Frische Blüten von Lavandula officinalis wurden einer Wasserdampfdestillation gemäß Ph. Eur unterzogen. Das so erhaltene etherische Öl wurde durch Destillation bei max. 120 °C und 10 bis 20 mbar weiter aufgereinigt.

### Beispiel 3: Kombinationspräparat in Form einer überzogenen Tablette

**Tabelle 2: Zusammensetzung von überzogenen Tabletten mit 500 mg Baldriantrockenextrakt und 80 mg Lavendelöl**

| Position | Inhaltsstoff | Menge pro Tablette [mg] | Menge pro Herstellungsansatz für 30.000 Tabletten [kg] |
|---|---|---|---|
| 1 | Baldrianwurzeltrockenextrakt | 500,0 | 15,00 |
| 2 | Gefälltes Siliciumdioxid | 100,0 | 3,00 |
| 3 | Lavendelöl | 80,0 | 2,40 |
| 4 | Mikrokristalline Cellulose | 200,0 | 6,00 |
| 5 | Croscarmellose Natrium | 40,0 | 1,20 |
| 6 | Gefälltes Siliciumdioxid | 20,0 | 0,60 |
| 7 | Magnesiumstearat | 5,0 | 0,15 |
| 8 | Hypromellose | 40,0 | 1,20 |
| 9 | Macrogol 1500 | 8,0 | 0,24 |
| 10 | Titandioxid E171 | 6,0 | 0,18 |
| 11 | Eisenoxid gelb E172 | 3,0 | 0,09 |
| 12 | Talkum | 3,0 | 0,09 |
| | | 1005,0 | 30,15 |

Zur Herstellung einer Tablette mit 500 mg festem Baldrianwurzeltrockenextrakt und 80 mg flüssigem Lavendelöl werden 15 kg Baldrianwurzeltrockenextrakt (Position 1) mit 3 kg gefälltem Siliciumdioxid (Position 2) intensiv gemischt. Anschließend wird zu dieser Mischung 2,4 kg Lavendelöl (Position 3) zugefügt und nochmals intensiv gemischt. Es entsteht eine pulverförmige Mischung ohne flüssige Anteile. Wird die Mischung aus Baldrianwurzeltrockenextrakt und Lavendelöl ohne gefälltes Siliciumdioxid hergestellt, scheidet sich das Lavendelöl in flüssiger Form ab und die Mischung ist für die Weiterverarbeitung zu Tabletten nicht geeignet.

Die Mischung aus Baldrianwurzeltrockenextrakt, Siliciumdioxid und Lavendelöl wird mit mikrokristalliner Cellulose (Füll- und Bindemittel; Position 4), Croscarmellose-Natrium (Zerfallsbeschleuniger; Position 5), gefälltem Siliciumdioxid (Fließregulierungsmittel; Position 6) versetzt und gemischt. Abschließend wird Magnesiumstearat (Schmiermittel; Position 7) zugefügt und nochmals für eine kurze Dauer von 5 Minuten gemischt.

Die Gesamtmischung wird auf einer Rundlauftablettenpresse zu ovalen Tabletten mit einer Einzelmasse von 945 mg verpresst. Zur Herstellung eines Überzuges werden die Bestandteile des Überzuges Hypromellose (Filmbildner, Position 8), Macrogol 1500 (Weichmacher Position 9), Titandioxid und Eisenoxid (Farbpigmente Position 10 und 11) sowie Talkum (Antiklebemittel, Position 12) in Wasser dispergiert und in einem Trommelcoater auf die Tabletten aufgetragen. Das Produkt sind gleichmäßig überzogene, gelb gefärbte Filmtabletten mit 500 mg Baldrianwurzeltrockenextrakt und 80 mg Lavendelöl zur oralen Einnahme.

### Beispiel 4: Kombinationspräparat in Form einer Weichkapsel

**Tabelle 3: Zusammensetzung einer Mischung zur Befüllung von Weich- oder Hartkapseln mit 250 mg Baldrianwurzeltrockenextrakt und 40 mg Lavendelöl**

| Position | Inhaltsstoff | Menge pro Kapsel [mg] | Menge pro Herstellungsansatz für 100.000 Kapseln [kg] |
|---|---|---|---|
| 1 | Hartfett | 230,0 | 23,0 |
| 2 | Hydriertes Sojaöl | 60,0 | 6,0 |
| 3 | Raffiniertes Rapsöl | 40,0 | 4,0 |
| 4 | Baldrianwurzeltrockenextra kt | 250,0 | 25,0 |
| 5 | Lavendelöl | 40,0 | 4,0 |
| | | 620,0 | 62,0 |

Zur Herstellung der Kapselfüllmasse werden Hartfett (Position 1), hydriertes Sojaöl (Position 2) und raffiniertes Rapsöl (Position 3) bei ca. 50 °C geschmolzen bzw. gelöst. Der Baldrianwurzeltrockenextrakt wird in der Schmelze homogen verteilt und abschließend das Lavendelöl zugegeben und homogen verteilt. Die fertige Kapselfüllmasse wird in einem dicht schließenden, temperierbaren Behälter unter Rühren aufbewahrt und in bei ca. 30 - 40 ° in Hartkapseln abgefüllt oder zur Befüllung von Weichkapseln verwendet. Durch die Einnahme von zwei Kapseln wird die gewünschte Menge von 500 mg Baldriantrockenextrakt und 80 mg Lavendel oral appliziert werden.

## Patentansprüche

1. Kombination umfassend einen Gehalt an Baldrianwurzelextrakt (Extrakt aus getrockneten Wurzeln von Valeriana officinalis) und Lavendelöl (Öl aus Blüten von Lavandula officinalis) zur oralen Verwendung bei der Behandlung oder Prophylaxe von Schlafstörungen, wobei der Baldrianwurzelextrakt durch Extraktion mit wässrigem Ethanol oder wässrigem Methanol hergestellt ist, wobei Ethanol und Wasser im Volumenverhältnis von 30/70 bis 90/10 oder Methanol und Wasser im Volumenverhältnis von 40/60 bis 55/45 bei der Extraktion eingesetzt werden.

2. Kombination zur Verwendung nach Anspruch 1, des Weiteren umfassend pharmazeutisch oder lebensmittelrechtlich akzeptable Hilfsstoffe.

3. Kombination zur Verwendung nach einem der Ansprüche 1 oder 2, wobei keine weiteren Pflanzenextrakte und/oder Pflanzenöle und/oder sonstige arzneilich wirksame Stoffe (Wirkstoffe) enthalten sind.

4. Kombination zur Verwendung nach einem der Ansprüche 1 bis 3, wobei als Baldrianwurzelextrakt ein Baldrianwurzeltrockenextrakt eingesetzt wird.

5. Kombination zur Verwendung nach Anspruch 4, wobei der Baldrianwurzeltrockenextrakt mindestens 0,25 Gew.-% Sesquiterpensäuren enthält.

6. Kombination zur Verwendung nach Anspruch 5, wobei das Gewichtsverhältnis von Valerensäure zu Acetoxyvalerensäure im Baldrianwurzeltrockenextrakt mindestens 2/1 beträgt.

7. Kombination zur Verwendung nach einem der Ansprüche 1 bis 6, wobei das Lavendelöl 20,0 Gew.-% bis 45,0 Gew.-% Linalool und 25,0 Gew.-% bis 47,0 Gew.-% Linalylacetat enthält.

8. Kombination zur Verwendung nach Anspruch 7, wobei das Lavendelöl darüber hinaus höchstens 1,0 Gew.-% Limonen, höchstens 2,5 Gew.-% 1,8-Cineol, 0,1 Gew.-% bis 5,0 Gew.-% 3-Octanon, höchstens 1,2 Gew.-% Campher, 0,1 Gew.-% bis 8,0 Gew.-% Terpinen-4-ol, mindestens 0,2 Gew.-% Lavandulylacetat, mindestens 0,1 Gew.-% Lavandulol und höchstens 2,0 Gew.-% alpha-Terpineol enthält.

9. Kombination zur Verwendung nach einem der Ansprüche 1 bis 8, wobei 40 bis 200 mg Lavendelöl und 80 bis 600 mg Baldrianwurzelextrakt pro Einzeldosis verwendet werden.

10. Kombination zur Verwendung nach Anspruch 9, wobei 40 bis 80 mg Lavendelöl und 250 bis 500 mg Baldrianwurzelextrakt pro Einzeldosis verwendet werden.

11. Kombination zur Verwendung nach einem der Ansprüche 1 bis 10, wobei die Schlafstörungen ausgewählt sind aus Ein- und Durchschlafstörungen, Störungen des Schlaf-Wach-Rhythmus, Schlafapnoe, nichtorganischer Insomnie, nichtorganischer Störung des Schlaf-Wach-Rhythmus, Schlafwandeln (Sonambulismus), Pavor nocturnus sowie Alpträume (Angstträume).

## Claims

1. Combination comprising a content of an valerian root extract (extract of dry roots of Valeriana officinalis) and lavender oil (oil of blossoms of Lavandula officinalis) for oral use in the treatment or prophylaxis of sleep disorders, wherein the valerian root extract is prepared by extraction with aqueous ethanol or aqueous methanol, wherein ethanol and water are used in a volume ratio of 30/70 to 90/10 or methanol and water are used in a volume ratio of 40/60 to 55/45 in the extraction.

2. Combination for use according to claim 1, further comprising additives which are pharmaceutically acceptable or acceptable under food law.

3. Combination for use according to claim 1 or 2, wherein no further plant extracts and/or plant oils and/or other pharmaceutically effective ingredients (drugs) are contained.

4. Combination for use according to any one of claims 1 to 3, wherein a dry valerian root extract is employed as the valerian root extract.

5. Combination for use according to claim 4, wherein the dry valerian root extract contains at least 0.25 wt.-% of ses-quiterpene acids.

6. Combination for use according to claim 5, wherein the weight ratio of valerenic acid to acetoxy valerenic acid in the dry valerian root extract is at least 2:1.

7. Combination for use according to any once of claims 1 to 6, wherein the lavender oil comprises 20.0 wt.-% to 45.0 wt.-% linalool and 25.0 wt.-% to 47.0 wt.-% of linalyl acetate.

8. Combination for use according to claim 7, wherein the lavender oil additionally comprises at most 1.0 wt.-% limonene, at most 2.5 wt.-% 1,8-cineol, 0.1 wt.-% to 5.0 wt.-% 3-octanone, at most 1.2 wt.-% camphor, 0.1 wt.-% to 8.0 wt.-% terpinen-4-ol, at least 0.2 wt.-% lavandulyl acetate, at least 0.1 wt.-% lavandulol and at most 2.0 wt.-% alpha-terpineol.

9. Combination for use according to any one of claims 1 to 8, wherein 40 to 200 mg lavender oil and 80 to 600 mg valerian root extract per single dose are used.

10. Combination for use according to claim 9, wherein 40 to 80 mg of lavender oil and 250 to 500 mg of valerian root extract per single dose are used.

11. Combination for use according to any one of claims 1 to 10, wherein the sleep disorders are selected from disorders of initiating and maintaining sleep, circadian rhythm sleep disorders, sleep apnoea, nonorganic insomnia, nonorganic disorder of the circadian rhythm, sleepwalking (somnambulism), pavor nocturnus as well as nightmares (anxiety dreams).

## Revendications

1. Combinaison comprenant une teneur en extrait de racine de valériane (extrait de racines séchées de *Valeriana officinalis*) et en huile de lavande (huile de fleurs de *Lavandula officinalis*) destinée à une utilisation orale lors du traitement ou de la prophylaxie de troubles du sommeil, dans laquelle l'extrait de racine de valériane est produit par extraction avec de l'éthanol aqueux ou du méthanol aqueux, dans laquelle l'éthanol et l'eau sont utilisés selon le rapport volumique allant de 30/70 à 90/10 ou le méthanol et l'eau sont utilisés selon le rapport volumique allant de 40/60 à 55/45 lors de l'extraction.

2. Combinaison utilisable selon la revendication 1, comprenant en outre des excipients acceptables pharmaceutiquement ou conformément à la législation alimentaire.

3. Combinaison utilisable selon l'une quelconque des revendications 1 ou 2, dans laquelle aucun extrait de plante et/ou aucune huile végétale et/ou aucune autre substance à efficacité médicinale (principes actifs) n'est contenu(e).

4. Combinaison utilisable selon l'une quelconque des revendications 1 à 3, dans laquelle un extrait sec de racine de valériane est utilisé en tant qu'extrait de racine de valériane.

5. Combinaison utilisable selon la revendication 4, dans laquelle l'extrait sec de racine de valériane contient au moins 0,25 % en poids d'acides sesquiterpéniques.

6. Combinaison utilisable selon la revendication 5, dans laquelle le rapport en poids entre l'acide valérénique et l'acide acétoxyvalérénique dans l'extrait sec de racine de valériane présente au moins une valeur de 2/1.

7. Combinaison utilisable selon l'une quelconque des revendications 1 à 6, dans laquelle l'huile de lavande contient 20,0 à 45,0 % en poids de linalol et 25,0 % à 47,0 % en poids d'acétate de linalyle.

8. Combinaison utilisable selon la revendication 7, dans laquelle l'huile de lavande contient par ailleurs au maximum 1,0 % en poids de limonène, au maximum 2,5 % en poids de 1,8-cinéol, 0,1 % en poids à 5,0 % en poids de 3-octanone, au maximum 1,2 % en poids de camphre, 0,1 % en poids à 8,0 % en poids de terpinène-4-ol, au moins 0,2 % en poids d'acétate de lavandulyle, au moins 0,1 % en poids de lavandulol et au maximum 2,0 % en poids d'alpha-terpinéol.

9. Combinaison utilisable selon l'une quelconque des revendications 1 à 8, dans laquelle 40 à 200 mg d'huile de lavande et 80 à 600 mg d'extrait de racine de lavande sont utilisés par dose unitaire.

10. Combinaison utilisable selon la revendication 9, dans laquelle 40 à 80 mg d'huile de lavande et 250 à 500 mg d'extrait de racine de valériane sont utilisés par dose unitaire.

11. Combinaison utilisable selon l'une quelconque des revendications 1 à 10, dans laquelle les troubles du sommeil sont choisis parmi les troubles de l'endormissement et de continuité du sommeil, les troubles du rythme sommeil-veille, le syndrome de l'apnée du sommeil, l'insomnie d'origine non organique, le trouble d'origine non organique du rythme sommeil-veille, le somnambulisme, la terreur nocturne ainsi que les cauchemars.
